# FASCICULE DE BREVET EUROPEEN

(11) **EP 3 947 343 B1**
(45) Date de publication et mention de la délivrance du brevet: **07.05.2025**
(21) Numéro de dépôt: 20723456.8
(22) Date de dépôt: 27.03.2020
(51) Int. Cl.: C07C 235/08, C09D 5/03

(54) **ADDITIFS DE RHÉOLOGIE À BASE DE DI- OU TRI-AMIDES HYDROXYLES ET DE LEURS MÉLANGES**
RHEOLOGIEADDITIVE AUF BASIS VON HYDROXYLIERTEN DI- ODER TRI-AMIDEN UND MISCHUNGEN DAVON
RHEOLOGY ADDITIVES BASED ON HYDROXYLATED DI- OR TRI-AMIDES AND MIXTURES THEREOF

(30) Priorité: 29.03.2019 FR 1903316
(43) Date de publication de la demande: 09.02.2022
(73) Titulaire: ARKEMA FRANCE, 92800 Puteaux (FR)
(72) Inventeur: COLESNIC, Dmitri, 75010 Paris (FR); LEROY, Vincent, 60550 VERNEUIL EN HALATTE (FR); LEPINAY, Laurent, 60550 VERNEUIL EN HALATTE (FR)
(74) Mandataire: Arkema Patent
(86) Numéro de dépôt international: PCT/FR2020/000071
(87) Numéro de publication internationale: WO 2020/201638

(56) Documents cités:
- EP-A2- 1 514 912
- WO-A1-2014/053774
- WO-A1-2015/011375
- JP-A- S61 234 919

## Description

La présente invention concerne un amide spécifique multifonctionnel (di- et triamide) convenable pour être utilisé comme organogélateur, en particulier comme additif de rhéologie et plus particulièrement dans des compositions de revêtements.

EP 1 514 912 décrit des triamides branchés d'acides gras non hydroxylés, à base de polyéther amines et utilisés comme agent vecteur de changement de phase dans les encres à changement de phase (appelés « hot melts inks ») avec comme fonction de faire passer l'encre de l'état solide à température ambiante à l'état liquide à température élevée dans des imprimantes à jet d'encre et permettant aux gouttelettes d'encre liquide de solidifier rapidement après leur jet à cette température. EP 1 514 912 ne suggère nullement l'utilisation de ces polyamides comme agent organogélateur ou agent thixotrope et ne suggère nullement des polyamides hydroxylés à base d'un mélange d'acide gras hydroxylés et de monoacides carboxyliques hydroxylés de chaîne plus courte.

Des diamides gras à base de diamines aliphatiques (sans segments polyéthers) à base d'acides gras hydroxylés sont connus comme agents organogélateurs et en particulier comme agents thixotropes.

WO 2014/053774 décrit des diamides d'acide gras hydroxylés en tant qu'agent organogélateur ou appelé aussi additif de rhéologie, en particulier dans des compositions de revêtements, de moulage, de mastics ou d'agent d'étanchéité ou de cosmétique.

WO 2015/011375 décrit des diamines d'acides gras comprenant dans sa structure à la fois des diamines cycloaliphatiques et aliphatiques avec un ratio molaire spécifique et l'utilisation de ces produits comme agent organogélateur ou comme additif de rhéologie, en particulier dans des compositions de revêtements, de moulage, de mastics ou d'agent d'étanchéité ou de cosmétique.

FR 2 993 885 décrit un diamide d'acide gras comprenant dans sa structure des acides carboxyliques hydroxylés spécifiques et l'utilisation de ce produit en tant qu'organogélateur dans des compositions de revêtements, de moulage, de mastics ou d'agent d'étanchéité.

Ces diamides connus ont besoin d'être micronisés à l'état de poudre et ensuite ont besoin d'une « activation » au préalable pour donner les performances rhéologiques requises. Le processus d'activation nécessite un cisaillement à haute vitesse et un chauffage allant parfois jusqu'à 100°C selon les produits. De plus, une durée minimale est requise, dépendante des conditions de température et de la polarité du système. D'autre part, ces additifs peuvent être peu compatibles avec certains liants de formulations réactives ou avec certains diluants ou plastifiants utilisés pour l'activation. Par conséquent, cette phase d'activation constitue un inconvénient particulier pour les poudres polyamides et les dérivés d'huiles de ricin hydrogénés, utilisées comme additifs dans ce domaine.

Il y a donc besoin de nouveaux polyamides gras permettant une mise en forme plus simple et plus facile (mise en forme sous forme d'écailles facilement solubles dans les plastifiants ou liants de formulations réactives d'application finale, sans besoin au préalable de préactivation) avec un plus large spectre de compatibilité avec les liants réactifs et plastifiants/diluants utilisés dans des formulations réactives telles que : polyéther terminé silane, polyuréthane terminé silane, polyuréthane terminé isocyanate, silicone, polysulfide, époxy, etc. Les polyamides (signifiant amides multi-fonctionnels et non polymères) requis comme additifs de rhéologie doivent conduire à des produits finaux en particulier des revêtements, joints de mastics ou joints d'agents de scellage qui ont un aspect de surface et d'esthétique améliorés qui sont transparents sans défaut de surface, ceci étant lié à la structure et composition spécifiques de ces polyamides visés.

La présente invention avec les nouveaux polyamides gras (amides gras multifonctionnels, en particulier di- et triamides) à base de polyamines primaires (di- et triamines) comprenant au moins un segment polyéther dans sa structure et en particulier à base de polyoxypropylène et à base d'acides gras comprenant au moins un acide gras hydroxylé en présence d'un acide hydroxylé de chaîne plus courte, permet de répondre aux besoins nouveaux définis ci-dessus.

Le premier objet de la présente invention concerne donc, un amide gras multi-fonctionnel qui est un diamide ou triamide gras ou leur mélange, à base d'une polyamine polyéther (diamine ou triamine) et d'au moins un acide gras saturé linéaire porteur d'un groupement hydroxy non terminal en présence d'un autre acide monocarboxylique plus court en C₂-C₁₀ porteur d'un groupement hydroxy.

Le deuxième objet de l'invention concerne une composition de formulation d'un liant organique, laquelle composition comprend au moins un liant organique et au moins un amide gras tel que défini selon la présente invention, en particulier en tant qu'additif rhéologique.

La présente invention couvre également l'utilisation d'au moins un amide gras tel que défini selon la présente invention, comme additif de rhéologie.

Finalement, l'invention couvre également le produit final obtenu qui résulte de l'utilisation d'au moins un amide gras tel que défini selon la présente invention, comme additif de rhéologie, en particulier comme agent thixotrope.

Ainsi, le premier objet de la présente invention est un amide gras multi-fonctionnel, qui est un diamide ou un triamide ou leur mélange et ledit amide gras est représenté par :
A) selon la formule (I) suivante :

   R[(-X-R1-NHCO-R2)_{n(1-y}] [(-X-R1-NHCO-R2')_{ny}] (I)

   avec
   - n étant 2 ou 3, de préférence 3
   - R(-X-R1-)ₙ étant le résidu de valence n d'une polyamine primaire R(-X-R1-NH₂)ₙ qui est une diamine ou une triamine primaire, avec chaque groupement d'amine primaire -NH₂ étant un groupement terminal porté par un segment bivalent de chaîne oligomère R1 choisi parmi polyéther et polyester qui est alkoxylé (polyester alcoxylé), de préférence polyéther et plus préférentiellement polyoxypropylène ou copolymères oxypropylène/oxyéthylène à majorité de motifs oxypropylène
   - R : résidu alkylène en C₃-C₁₀ de valence n issu d'un polyol R(OH)ₙ ou d'une polyamine R(NH₂)ₙ ou R(NH-R3)ₙ, de préférence d'un polyol R(OH)ₙ
   - X : O, NH ou NR3, de préférence O
   - R2 étant le résidu gras, sans groupement carboxy, en C₁₂-C₅₂, de préférence en C₁₆-C₃₆, plus préférentiellement en C₁₆-C₂₄ d'acide gras hydroxylé R2CO₂H, en particulier saturé et linéaire
   - R2' étant le résidu d'acide monocarboxylique R2'CO₂H, en C₂ à C₁₀, de préférence de C₂ à C₈, plus préférentiellement en C₂ à C₆, et porteur d'au moins un groupement hydroxy et de préférence d'au moins deux groupements hydroxy avec y représentant la fraction molaire de R2'CO₂H par rapport à la somme R2CO₂H + R2'CO₂H (R2'/(R2+R2')) dans ledit diamide, avec y variant de 0,05 à 0,50, de préférence de 0,10 à 0,40, avec R2CO₂H et/ou R2'CO₂H pouvant être des mélanges d'acides respectifs,
   - R3 étant un substituant alkyl en C₁-C₂
   ou (ledit amide gras est représenté) par :
   B) selon la formule (II) suivante dans le cas où ledit amide est un diamide :

   (R2CONH)_{(1-y)}-R'-O-[CH₂-CH(R4)-O]ₓ-CH₂-CH(R4)-(NHCOR'2)_{y} (II)

   avec R' étant le résidu du monopropylène glycol sans OH : -CH(CH₃)-CH₂-
   et
   x étant le nombre d'unités oxyalkylène -CH₂-CH(R4)-O- et pouvant varier de 5 à 45 de préférence de 5 à 40 et plus préférentiellement de 5 à 35.
   R2 et R'2 et « y » étant définis comme dans la formule (I) ci-dessus et R4 étant H ou méthyl avec l'unité répétitive oxyalkylène -CH₂-CH(R4)-O- étant éthoxy pour R4 étant H et propoxy pour R4 étant méthyl ou R4 correspond à un mélange éthoxy/propoxy et de préférence R4 est méthyl avec ladite unité oxyalkylène étant propoxy et ledit amide ayant un point de fusion signifiant température de fusion, mesurée par DSC après deux passages, 10°C/min allant de 10 à 110°C, de préférence de 20 à 100°C.

Le terme « point de fusion » correspond à la température de fusion mesurée par calorimétrie différentielle à balayage (DSC) à une vitesse de chauffe de 10°C/min. Cette température est la température qui correspond au pic de fusion enregistré par DSC à la vitesse de chauffe précisée.

Concernant le résidu R, ledit alkylène en C₃-C₁₀ en plus des liaisons carbone-carbone peut comprendre un pont éther -O- dans le cas d'un résidu de polyol ou un pont -NH- dans le cas d'un résidu de polyamine.

Dans le cas où X : N, comme ceci apparaît évident à partir des formules précisées ci-haut de polyamines à résidu R, cela signifie que N représente -NH- et -N(R3)-.

Concernant la signification de R2CO₂H, il s'agit d'acides gras linéaires hydroxylés avec l'hydroxy non terminal. Ces acides gras linéaires sont à chaîne grasse linéaire en C₁₂-C₅₂, de préférence en C₁₆-C₃₆, plus préférentiellement en C₁₆-C₂₄, en particulier constituée exclusivement de liaisons C-C et donc sans groupements esters dans cette chaîne linéaire. Cette définition exclut donc de la définition de R2CO₂H tout polyester ou oligoester issu de l'auto polycondensation d'un acide gras hydroxylé.

Dans le cas des acides monocarboxyliques R2'CO₂H, le terme « en C₂ -C₁₀, de préférence en C₂-C₈, plus préférentiellement en C₂-C₆ » signifie qu'il s'agit de la longueur de la chaîne de R2' exprimé en nombre d'atomes de carbone enchainés (-C-C-), sans tenir compte des substituants latéraux.

Selon une option spécifique l'acide R2CO₂H est un mélange d'acide gras hydroxylés et/ou l'acide R2'CO₂H est un mélange d'acides monocarboxyliques hydroxylés de chaîne plus courte (que les acides gras hydroxylés) comme définis selon l'invention.
Comme exemples convenables de polyamines primaires répondant à la formule R(-X-R1-NH₂)ₙ qui sont des diamines ou des triamines primaires comme définies ci-dessus, on peut citer les suivantes :
- comme diamine (n = 2) ou triamine (n = 3) : une diamine primaire avec les deux fonctions d'amine primaire portées par un segment polyéther ou un segment de polyester alcoxylé (polyester-polyéther) ou une triamine avec 3 fonctions amine primaire portées par 3 segments polyéther ou polyester alcoxylé (polyester-polyéther), ledit segment polyéther ou polyester alcoxylé pour une diamine ou l'ensemble des 3 segments polyéthers ou polyesters alcoxylés dans le cas d'une triamine, ayant une masse moléculaire moyenne en nombre Mn allant de 500 à 3000. En particulier, il s'agit des diamines et triamines primaires polyéthers et plus particulièrement des polyoxypropylène diamines et triamines primaires comme les Jeffamine^{®} diamines et triamines commercialisées par Hunstmann avec comme exemples plus particuliers convenables la Jeffamine^{®} D-2000 (diamine primaire avec un segment polyoxypropylène porteur de 2 groupements amine primaire avec un nombre d'unités oxypropylène de 33) ou la Jeffamine^{®} T-3000 (triamine primaire avec 3 segments polyoxypropylène et un nombre total d'unités oxypropylène de 50). D'autres amines peuvent encore être utilisées : Jeffamine^{®} D-400, Jeffamine^{®} D-2010, Jeffamine^{®} T-403, Jeffamine^{®} T-5000, etc.

En principe, lesdits polyéther diamines ou polyéther triamines convenables pour la préparation des diamides et triamides selon la présente invention peuvent être obtenus à partir de précurseurs polyéther polyols correspondants (diols ou triols respectifs) par amination réductrice des fonctions OH terminales en présence d'un catalyseur comme décrit dans US 4766245 ou GB 2175910.

Concernant les polyéthers polyols (diols ou triols) précurseurs respectifs de polyéthers diamines ou triamines, ils peuvent être obtenus par polymérisation anionique en milieu basique de l'oxyde d'alkylène correspondant (oxyde d'éthylene pour polyoxyéthylène diol/triol, oxyde de propylène pour polyoxypropylène diol/triol) ou d'un mélange desdits oxydes d'alkylènes en présence d'un amorceur polyol alcoolate avec OH primaires qui est respectivement diol ou triol (à OH primaires) ou un amorceur polyamine respectivement diamine et triamine (suivant la fonctionnalité dudit polyéther : diol ou triol). Comme exemple d'amorceur diol, on peut citer l'éthylène glycol, diéthylène glycol, propylène 1,3-glycol, butylène 1,4-glycol. Comme exemple d'amorceur triol, on peut citer le triméthylol propane.

Dans le cas d'amorceurs bivalents (deux fonctions alcoolates primaires ou deux fonctions amines), ils conduisent à une structure symétrique avec l'amorceur incorporé au milieu de la chaîne (via liaison éther -O- ou -NH-) et avec départ d'une chaîne polyéther pour chaque fonction alcoolate ou amine de l'amorceur utilisé.

Dans le cas d'un amorceur trivalent (triol alcoolate primaire ou triamine), chaque alcoolate ou amine est le point de départ d'une chaîne polyéther avec comme résultat 3 chaînes polyéther portées par une molécule dudit amorceur dont le résidu correspond à R dans la formule définie ci-haut pour l'amide selon l'invention.

Dans le cas particulier de polyéthers diamines, les précurseurs polyéthers diols peuvent également être obtenus par polymérisation anionique de l'alkylène oxyde ou d'un mélange correspondant d'alkylènes oxydes (par exemple oxyde d'éthylène ou oxyde de propylène ou mélange d'oxyde d'éthylène et d'oxyde de propylène pour respectivement polyoxyéthylène diols, polyoxypropylène diols et copolymères (oxyéthylene-oxypropylène) diols) à partir d'un amorceur alcoolate primaire monovalent porteur sur un carbone secondaire dudit amorceur un OH (OH secondaire ne réagissant pas pour ouvrir l'oxyde d'alkylène). Dans un tel cas, une seule chaîne polyéther est formée à partir de l'alcoolate primaire l'autre bout OH (secondaire) de l'amorceur restant libre et inchangé et ainsi avec le polyéther formé étant un diol (précurseur du polyéther diamine par conversion des OH terminaux en NH2 comme cité ci-haut). Un exemple de diol amorceur monovalent (1 seul OH primaire) est le monopropylène glycol sous forme d'alcoolate primaire comme ci-dessous:

HO-CH(CH₃)-CH₂O ⁻

Dans le cas plus particulier de polyéther diamines à base de polyoxypropylène, le monopropylène glycol agit comme amorceur monovalent avec hydroxyle secondaire non affecté durant la polymérisation de l'oxyde de propylène (amorçage par ouverture du cycle par attaque de l'amorceur anionique alcoolate sur l'atome de carbone le moins riche en électrons (-CH₂-) de l'oxyde de propylène et ensuite propagation en chaîne, conduisant au polyoxypropylène diol de formule suivante qui est porteur de deux OH secondaires :

HO-CH(CH₃)-CH₂-O-(CH₂-CH(CH₃)-O)ₓ-CH₂-CH(CH₃)-OH

Après conversion des hydroxyles secondaires terminaux (par amination réductrice catalysée sous pression NH₃ comme décrit dans US 4766245 ou GB2175910), la polyoxypropylène diamine de formule suivante peut être obtenue :

H₂N-CH(CH₃)-CH₂O-(CH₂-CH(CH₃)-O)ₓ-CH₂-CH(CH₃)-NH₂

Comme exemple d'une telle polyoxypropylène diamine, on peut citer la Jeffamine^{®} D2000 commercialisé par Hunstman.

Comme acides gras saturés linéaires hydroxylés R2CO₂H (avec R2 porteur d'un OH non terminal) tels que définis selon l'invention, on peut utiliser un hydroxy acide gras parmi l'acide 12-hydroxy stéarique (12-HSA), l'acide 9-hydroxy stéarique (9-HSA), l'acide 10-hydroxystéarique (10-HSA), l'acide 14-hydroxy eicosanoïque (14-HEA) ou leurs mélanges.

Comme acides plus courts R2'CO2H en C₂-C₁₀, on peut utiliser l'acide 2,2-bis (hydroxyméthyl) propionique, acide 2,2-bis (hydroxyméthyl) butyrique, acide hydroxy-acétique (ou acide glycolique), acide 2-hydroxy propionique (acide lactique), acide 2-hydroxy-3-(3-pyridyl) propionique, acide 3-hydroxy butyrique, acide 2-hydroxy butyrique, acide 2-méthyl-2-hydroxy butyrique, acide 2-éthyl-2-hydroxy butyrique, acide hydroxy pentanoïque, acide hydroxy hexanoïque, acide hydroxy heptanoïque, acide hydroxy octanoïque, acide hydroxy nonanoïque, acide hydroxy décanoïque et leurs mélanges ; de préférence l'acide 2,2-bis (hydroxyméthyl) propionique, acide 2,2-bis (hydroxyméthyl) butyrique, acide hydroxy-acétique (ou acide glycolique), acide 2-hydroxy propionique (acide lactique), acide 2-hydroxy-3-(3-pyridyl) propionique, acide 3-hydroxy butyrique, acide 2-hydroxy butyrique, acide 2-méthyl-2-hydroxy butyrique, acide 2-éthyl-2-hydroxy butyrique, acide hydroxy pentanoïque, acide hydroxy hexanoïque, acide hydroxy heptanoïque, acide hydroxy octanoïque et leurs mélanges ; plus préférentiellement l'acide 2,2-bis (hydroxyméthyl) propionique, acide 2,2-bis (hydroxyméthyl) butyrique, acide hydroxy-acétique (ou acide glycolique), acide 2-hydroxy propionique (acide lactique), acide 2-hydroxy-3-(3-pyridyl) propionique, acide 3-hydroxy butyrique, acide 2-hydroxy butyrique, acide 2-méthyl-2-hydroxy butyrique, acide 2-éthyl-2-hydroxy butyrique, acide hydroxy pentanoïque, acide hydroxy hexanoïque et leurs mélanges.

L'amide gras selon l'invention représenté par A selon formule (I) a de préférence une masse moléculaire moyenne en nombre Mn mesurée par GPC dans le THF en équivalents polystyrène (calibration par étalons de polystyrène) qui varie pour :
- n = 2 (un diamide) de 800 à 4000, de préférence de 1000 à 3800
- n = 3 (un triamide) de 1000 à 6000, de préférence de 2000 à 5500.

L'amide gras selon l'invention représenté par B selon formule (II) qui est un diamide gras a de préférence la même plage de masse moléculaire moyenne en nombre Mn mesurée par GPC dans le THF en équivalents polystyrène (calibration par étalons de polystyrène) que le diamide (n = 2) représenté par option A) selon formule (I), c'est-à-dire : de 800 à 4000, de préférence de 1000 à 3800.

Selon une option préférée pour l'amide gras représenté par l'option A) selon formule (I), ledit segment de chaîne oligomère R1 est un segment de chaîne polyéther.

Selon une option plus particulièrement préférée, ledit segment de chaîne oligomère R1 est un segment de chaîne polyoxypropylène.

Ledit segment de chaîne oligomère R1 peut avoir une masse moléculaire moyenne en nombre Mn allant de 400 à 2000, de préférence de 500 à 1500.

Selon une option spécifique de l'invention ledit amide gras est un diamide gras représenté par l'option B) selon formule (II) comme définie ci-haut.

Selon une option préférée, ledit acide gras hydroxylé R2CO₂H est sélectionné parmi l'acide 12-hydroxy stéarique (12-HSA) ; l'acide 9- ou 10- hydroxy stéarique (9-HSA ou 10-HSA), de préférence un mélange d'acide 9- et 10- hydroxy stéarique ; l'acide 14-hydroxy eicosanoïque (14-HEA) et leurs mélanges par deux. L'acide R2CO₂H hydroxylé le plus préféré est l'acide 12-hydroxystéarique.

De préférence plus particulière, ledit monoacide carboxylique R2'CO₂H est sélectionné parmi : acide 2,2-bis (hydroxyméthyl) propionique, acide 2,2-bis (hydroxyméthyl) butyrique, acide hydroxy-acétique (ou acide glycolique), acide 2-hydroxy propionique (acide lactique), acide 2-hydroxy-3-(3-pyridyl) propionique, acide 3-hydroxy butyrique, acide 2-hydroxy butyrique, acide 2-méthyl-2-hydroxy butyrique, acide 2-éthyl-2-hydroxy butyrique.

Selon une autre option particulière de l'invention, ledit amide est un diamide selon A) ou B) ou un triamide selon A), avec le ratio « y/(1-y) » variant de 1/20 à 1/2 et de préférence de 1/10 à 4/10.

Selon une autre option alternative, ledit amide est un diamide selon A) ou B) avec le ratio y/(1-y) variant de 1/10 à 1/2 et de préférence de 1/10 à 4/10.

Selon une option particulière ledit amide est un triamide selon A) avec deux résidus (R2) issus d'acide gras hydroxylé R2CO₂H et un (R2') issu d'acide R2'CO₂H.

Plus particulièrement et en alternative, ledit amide est un diamide représenté selon l'option A) et représenté par la formule (I) ou selon option B) et représenté par la formule (II) comme définies ci-avant.

Le deuxième objet de l'invention concerne une composition de formulation d'un liant organique caractérisée en ce qu'elle comprend :
a) au moins un liant organique et
b) au moins un amide gras tel que défini ci-dessus selon l'invention, en particulier en tant qu'additif rhéologique.

Plus particulièrement, dans ladite composition de formulation de liant, ledit liant a) est sélectionné parmi : les résines polysiloxanes terminées par des groupements silanes bloqués, les résines polyéthers terminées par des groupements silanes bloqués, les résines polysulfides terminées par des groupements silanes bloqués, les résines prépolymères polyuréthanes terminés par des groupements isocyanates, les résines PVC pour plastisols, les résines époxy porteuses de groupements époxy.

Ladite composition peut comprendre en plus de a) et b) et en fonction dudit liant un plastifiant ou un diluant réactif comme défini ci-dessous :
c) un plastifiant pour les résines polysiloxanes, prépolymères polyuréthanes et résines PVC pour plastisols ou
d) un diluant réactif parmi monomères époxydés pour les résines époxy et en option
e) pour les systèmes deux composants un durcisseur pour les résines époxy ou polyuréthane.

Plus particulièrement dans ladite composition selon l'invention, ledit amide gras est utilisé comme additif rhéologique qui est un agent thixotrope.

Dans ladite composition, ledit liant organique a) peut être sélectionné parmi une résine polysiloxane, une résine prépolymère polyuréthane ou une résine PVC pour plastisol et ledit plastifiant peut être sélectionné parmi : les phtalates, les adipates, les trimellitates, les sebacates, les benzoates, les citrates, les phosphates, les époxydes, les polyesters, les esters alkyl-sulfonates et non-phtalates substituts de phtalates.

Ladite composition peut être une composition de formulation de mastic transparent ou non. En particulier, il peut s'agir d'une composition de formulation de mastic transparent.

Un autre objet de l'invention couvre l'utilisation d'au moins un amide gras tel que défini ci-dessus selon l'invention où ledit amide est utilisé comme additif de rhéologie.

Dans ladite utilisation, ledit additif de rhéologie peut être utilisé comme agent thixotrope.

Plus particulièrement, ladite utilisation peut être dans des compositions de revêtements, d'adhésifs, de plastisols PVC ou de mastics, de préférence des compositions de plastisols PVC et des compositions de mastics.

Une autre utilisation particulière est dans des compositions de plastisols PVC.

Une autre utilisation particulière est dans des compositions de mastics réticulables par l'humidité à base de résines polysiloxanes terminées par des groupements silanes bloqués, les résines polyéthers terminées par des groupements silanes bloqués, les résines polysulfides terminées par des groupements silanes bloqués, en particulier silanes bloqués par groupements alkoxy ou des résines prépolymères polyuréthanes terminées par des groupements isocyanates.

Une autre utilisation particulière est dans des compositions des mastics réticulables par l'humidité, transparents ou non.

Finalement, l'invention couvre un produit final qui peut être un revêtement en particulier revêtement de plastisol PVC ou un joint d'adhésif ou un joint de mastic, qui résulte de l'utilisation d'au moins un amide gras tel que défini ci-dessus selon l'invention, comme additif de rhéologie et en particulier comme agent thixotrope.

Les exemples suivants de la partie expérimentale ci-dessous sont présentés à titre d'illustration de l'invention et de ses performances et ne limitent nullement sa portée.

### PARTIE EXPERIMENTALE

### 1) Matières premières utilisées et codes

### Voir tableau 1 ci-dessous

Tableau récapitulatif des matières premières utilisées en synthèse et en formulations

**[Tableau 1]**

| **Produit utilisé** | **Nom chimique** | **Fonction** | **Fournisseur** |
|---|---|---|---|
| 12HSA | Acide 12-hydroxy stéarique | Acide gras hydroxylé | Jayant Agro |
| Stéarine | Acide stéarique | Acide gras non hydroxylé, selon R2CO₂H | Sogis |
| bMBA | Acide 2,2-bis(hydroxyméthyl) butyrique | Acide court hydroxylé selon R2'CO₂H | Sigma Aldrich |
| JEFFAMINE^{®} T-3000 Polyétheramine | Polyétheramine Jeffamine^{®} T-3000 | Polyoxypropylène triamine (primaire) avec globalement ∼50 unités oxypropylène (OP) | Hunstmann |
| JEFFAMINE^{®} D-2000 Polyétheramine | Polyétheramine Jeffamine^{®} D-2000 | Polyoxypropylène diamine (primaire) avec -33 unités OP | Hunstmann |
| HCO (en écailles) | Huile de ricin hydrogéné | Additif de rhéologie de référence | Gokul Agro |
| Crayvallac^{®} Antisettle CVP (poudre micronisée) | Huile de ricin hydrogéné | Additif de rhéologie de référence | Arkema |
| Diamide gras standard | 12HSA-HMDA-12HSA | Additif de rhéologie diamide de référence pour comparaison | / |
| MS Polymer^{®} S203H | Polyéther silylé | Résine de formulation applicative | Kaneka |
| Jayflex^{®} DIUP | Diisoundecylphtalate | Plastifiant pour formulation | BASF |

Pour des raisons de clarté, nous allons utiliser les abréviations suivantes :
- 12HSA : Acide 12-hydroxy stéarique
- SA : Acide stéarique
- HMDA : Hexaméthylènediamine
- D2000 : Polyétheramine Jeffamine^{®} D-2000
- T3000 : Polyétheramine Jeffamine^{®} T-3000
- bMBA : Acide 2,2-bis(hydroxyméthyl) butyrique

### 2) Exemples

### Exemple A selon l'invention - T3000-(12HSA-bMBA)₃

Dans un ballon de 1 litre équipé d'un thermomètre, d'un Dean Stark, d'un condensateur et d'un agitateur, 231,40 g de Jeffamine^{®} T-3000 (0,078 moles, 1 éq), 63,11 g d'acide 12-hydroxystéarique (0,199 moles, 2,55 éq) et 5,2 g d'acide 2,2-bis(hydroxyméthyl) butyrique (0,035 moles, 0,45 éq) sont ajoutés. Le mélange est chauffé à 180°C sous atmosphère inerte. L'eau éliminée s'accumule dans le Dean Stark dès 150°C. La réaction est contrôlée par l'indice d'acide et d'amine. Lorsque les indices d'acide et d'amine sont respectivement inférieurs à 6, la réaction est arrêtée. Le mélange réactionnel est refroidi à 140°C et est déchargé dans un moule siliconé. Une fois refroidi à la température ambiante, le produit est transformé en écailles.

### Exemple B selon l'invention - D2000-(12HSA-bMBA)₂

Dans un ballon de 1 litre équipé d'un thermomètre, d'un Dean Stark, d'un condensateur et d'un agitateur, 232,3 g de Jeffamine^{®} D-2000 (0,115 moles, 1 éq), 62,12 g d'acide 12-hydroxystéarique (0,196 moles, 1,7 éq) et 5,11 g d'acide 2,2-bis(hydroxyméthyl) butyrique (0,034 moles, 0,3 éq) sont ajoutés. Le mélange est chauffé à 180°C sous atmosphère inerte. L'eau éliminée s'accumule dans le Dean Stark dès 150°C. La réaction est contrôlée par l'indice d'acide et d'amine. Lorsque les indices d'acide et d'amine sont respectivement inférieurs à 6, la réaction est arrêtée. Le mélange réactionnel est refroidi à 140°C et est déchargé dans un moule siliconé. Une fois refroidi à la température ambiante, le produit est transformé en écailles.

### Exemple C comparatif - T3000-(SA)₃

Dans un ballon de 1 litre équipé d'un thermomètre, d'un Dean Stark, d'un condensateur et d'un agitateur, 313,6 g de Jeffamine^{®} T-3000 (0,10 moles, 1 éq) et 86,4 g d'acide stéarique (0,3 moles, 3 éq) sont ajoutés. Le mélange est chauffé à 180°C sous atmosphère inerte. L'eau éliminée s'accumule dans le Dean Stark dès 150°C. La réaction est contrôlée par l'indice d'acide et d'amine. Lorsque les indices d'acide et d'amine sont respectivement inférieurs à 6, la réaction est arrêtée. Le mélange réactionnel est refroidi à 140°C et est déchargé dans un moule siliconé.

### Exemple D comparatif - D2000-(SA)₂

Dans un ballon de 1 litre équipé d'un thermomètre, d'un Dean Stark, d'un condensateur et d'un agitateur, 312,2 g de Jeffamine^{®} D-2000 (0,15 moles, 1 éq) et 87,8 g d'acide stéarique (0,3 moles, 2 éq) sont ajoutés. Le mélange est chauffé à 180°C sous atmosphère inerte. L'eau éliminée s'accumule dans le Dean Stark dès 150°C. La réaction est contrôlée par l'indice d'acide et d'amine. Lorsque les indices d'acide et d'amine sont respectivement inférieurs à 6, la réaction est arrêtée. Le mélange réactionnel est refroidi à 140°C et est déchargé dans un moule siliconé.

### 3) Etude du pouvoir gélifiant des organogélateurs

Dans cette étude nous comparons la capacité des additifs de rhéologie testés, à former un gel dans une formulation simplifiée contenant uniquement un plastifiant classique (Jayflex^{®} DIUP) utilisé dans les formulations PVC plastisols.

La préparation des formulations se fait à l'aide d'un mélangeur dit planétaire de laboratoire (type Molteni^{®} EMD 1) muni d'un disque de dispersion et d'un racleur permettant de mélanger des produits à haute viscosité, mais également des poudres dans des systèmes non fluides. Il est équipé d'une pompe à vide permettant d'éviter l'entrée d'humidité lors de la dispersion. La température à l'intérieur du Molteni^{®} EMD 1 est relevée par une sonde fixée au racleur et peut être régulée grâce à un bain.

Les formulations simplifiées testées/comparées, sont présentées au tableau 2 ci-dessous avec comme plastifiant identique étant Jayflex^{®} DIUP et comme additif de rhéologie variable testé et comparé, les amides cités ou autres produits de référence cités.

### Composition des formulations simplifiées

**[Tableau 2]**

| **Formulation** | **Composant** | **% poids** |
|---|---|---|
| F1 | Plastifiant Jayflex^{®} DIUP | 95 |
| | **Amide Exemple A** | 5 |
| F2 | Plastifiant Jayflex^{®} DIUP | 95 |
| | **Amide Exemple B** | 5 |
| F3 | Plastifiant Jayflex^{®} DIUP | 95 |
| | **Amide Exemple C** | 5 |
| F4 | Plastifiant Jayflex^{®} DIUP | 95 |
| | **Amide Exemple D** | 5 |
| F5 | Plastifiant Jayflex^{®} DIUP | 95 |
| | **12HSA-HMDA-12HSA** | 5 |
| F6 | Plastifiant Jayflex^{®} DIUP | 95 |
| | **HCO** | 5 |
| F7 | Plastifiant Jayflex^{®} DIUP | 95 |
| | **Crayvallac^{®} Antisettle CVP** | 5 |

L'additif de rhéologie est introduit dans le plastifiant et le mélange est porté à la température d'incorporation (cf Tableau 3) et dispersé pendant 5 minutes. A la fin de la dispersion, le mélange est refroidi à température ambiante et le comportement du gel est étudié visuellement (voir tableau 3 ci-dessous).

### Comportement et aspect du gel en fonction de la température d'incorporation

**[Tableau 3]**

| **Formulation** | **Température** | **Comportement du gel** | **Aspect** |
|---|---|---|---|
| F1 | 60°C** | Gel fort | Transparent |
| | 80°C** | Gel fort | Transparent |
| F2 | 60°C** | Gel fort | Transparent |
| | 80°C** | Gel fort | Transparent |
| F3 | 60°C** | Liquide | Transparent |
| | 80°C** | Liquide | Transparent |
| F4 | 60°C** | Liquide | Transparent |
| | 80°C** | Liquide | Transparent |
| F5 | 60°C* | Gel faible | Opaque |
| | 80°C* | Gel faible | Opaque |
| | 100°C** | Gel faible | Opaque |
| F6 | 60°C* | Gel faible (présence de grains) | Opaque |
| | 80°C** | Gel faible | Opaque |
| F7 | 60°C* | Gel fort | Opaque |
| | 80°C** | Gel faible (légère synérèse) | Opaque |

| | | | |
|---|---|---|---|
| *solubilisation partielle ; **solubilisation totale | | | |

Les résultats des tests de gel montrent que les produits selon l'invention (amides selon **exemples A et B**) forment des gels, tandis que les produits comparatifs sont sous forme liquide. Ainsi, le composé de l'**exemple C** décrit notamment dans EP 1 514 912 A2 ne permet pas l'obtention du gel (cf Formulation F3), ce qui montre fortement que la présence du groupement hydroxy, apportant des liaisons H, est indispensable à la formation de l'assemblage supramoléculaire et du réseau tridimensionnel (3D) de fibres formées.

Le comportement des agents organogélateurs peut être également influencé par la structure initiale de l'amine utilisée. Ainsi, en comparant l'organogélateur décrit dans WO 2014/053774A1 (**12HSA-HMDA-12HSA**) avec le composé de l'**Exemple B** selon l'invention, on peut remarquer une différence importante de force de gel. Notamment si on remplace l'amine aliphatique par une amine polyéther, le pouvoir gélifiant augmente, permettant, de plus, l'obtention d'un gel transparent. A noter que pour être entièrement solubilisé, le composé **12HSA-HMDA-12HSA** (cf Formulation F5) nécessite des températures plus importantes que les produits selon l'invention.

De plus, les performances des gels peuvent être reliées à la nature physique de l'additif de rhéologie. Par conséquent, on observe pour les formulations F6 et F7 dans un premier temps à température d'incorporation constante (60°C) de l'additif de rhéologie, une différence de force de gel. A savoir, si l'additif est sous forme d'écailles (cf Formulation F5), la force de gel va diminuer, ce qui pourrait être expliqué probablement par une incorporation incomplète du produit dans la formulation dû au manque de solubilité. Par ailleurs, des grains ont pu être observés, ce qui pourrait corroborer cette hypothèse.

Egalement, on peut observer dans le cas où l'additif est sous forme de poudre et incorporé à une température plus élevée (80°C dans F7) que sa température d'incorporation optimale (60°C dans F7), la force de gel va diminuer. Ceci montre en plus une sensibilité à la température, due probablement à la sur-solubilisation du produit. Il est donc important pour les produits standards de bien respecter une fenêtre de température d'incorporation pour que l'organogélateur soit efficace.

En ce qui concerne les formulations F1 et F2 contenant les produits selon l'invention, on peut remarquer la formation d'un fort gel, ceci indépendamment de la température d'incorporation. Il faut mentionner qu'aux températures étudiées, l'additif de rhéologie est entièrement solubilisé. De plus, les formulations présentent un aspect totalement transparent.

### 4) Evaluation des performances rhéologiques dans une formulation simplifiée de mastic hybride

Dans cette étude, sont comparées les performances rhéologiques des additifs d'une formulation simplifiée de mastic hybride comprenant comme plastifiant : Jayflex^{®} DIUP et comme additif de rhéologie le produit comparé cité.

### Composition des formulations simplifiées de mastic hybride

**[Tableau 4]**

| **Formulation** | **Composant** | **% en poids** | **Fonction** |
|---|---|---|---|
| F8 | Plastifiant Jayflex^{®} DIUP | 47,5 | Plastifiant |
| | MS-Polymer^{®} S 203 H | 47,5 | Résine |
| | **Amide selon exemple A** | 5 | Additif de rhéologie |
| F9 | Plastifiant Jayflex^{®} DIUP | 47,5 | Plastifiant |
| | MS-Polymer^{®} S 203 H | 47,5 | Résine |
| | **Crayvallac Antisettle CVP** | 5 | Additif de rhéologie |
| F10 | Plastifiant Jayflex^{®} DIUP | 47,5 | Plastifiant |
| | MS-Polymer^{®} S 203 H | 47,5 | Résine |
| | **Amide selon exemple B** | 5 | Additif de rhéologie |
| F11 | Plastifiant Jayflex^{®} DIUP | 47,5 | Plastifiant |
| | MS-Polymer^{®} S 203 H | 47,5 | Résine |
| | **12HSA-HMDA-12HSA** | 5 | Additif de rhéologie |

Les formulations sont préparées à l'aide du mélangeur Molteni^{®} EMD 1. Dans une première étape et dans les proportions indiquées (tableau 4), la résine et le plastifiant sont ajoutés et homogénéisés. L'additif est pesé et ajouté ensuite lors de la seconde étape. Ainsi, le mélange réactionnel, qui est maintenu sous vide pendant les phases de mélanges, est porté à 80°C pendant 5 minutes. A la fin de cette phase, le mélange est refroidi à 25°C et déchargé. Les performances de ces formulations sont présentées au tableau 5 ci-dessous.

### Performances rhéologiques

**[Tableau 5]**

| **Formulation** | **Viscosité à 0.1 s-1 (Pa.s)** | **Viscosité à 100 s-1 (Pa.s)** | **Indice thixotropique** | **Seuil d'écoulement (Pa)** | **Aspect** |
|---|---|---|---|---|---|
| F8 | 717 | 3,50 | 205 | 73 | Transparent |
| F9 | 296 | 6,06 | 49 | 4,4 | Opaque |
| F10 | 313 | 2,78 | 113 | 30 | Transparent |
| F11 | 47 | 2,21 | 21 | 3,1 | Opaque |

L'additif de rhéologie triamide de l'**exemple A** selon l'invention se montre bien plus efficace en terme de performances rhéologiques (cf Formulation F8), comparé à l'additif poudre standard Crayvallac^{®} Antisettle CVP (cf Formulation F9). Quant au produit diamide de l'**exemple C,** il présente également des performances rhéologiques supérieures (cf Formulation F10) à celle utilisant le composé sous forme de poudre **12HSA-HMDA-12HSA** (cf Formulation F11).

De plus, les produits selon l'invention ne nécessitent pas de processus spécifique de mise en oeuvre pour développer la rhéologie (gel), comme il est nécessaire dans le cas pour les additifs classiques sous forme de poudres à base de dérivés d'huile de ricin hydrogénée.

Par ailleurs, les produits selon l'invention étant sous forme d'écailles on s'affranchit ainsi des problématiques rencontrées avec l'utilisation des poudres (micronisation, manipulation, toxicité, etc.). Il est à noter également que ces produits permettent d'obtenir des formulations de mastic MS totalement transparents.

## Revendications

1. Amide gras multi-fonctionnel, **caractérisé en ce qu'**il s'agit d'un diamide ou d'un triamide ou de leur mélange et que ledit amide gras est représenté par:
A) selon la Formule (I) suivante :
R[(-X-R1-NHCO-R2)_{n(1-y)}] [(-X-R1-NHCO-R2')_{ny}] (I)
avec
- n étant 2 ou 3, de préférence 3
- R(-X-R1-)ₙ étant le résidu de valence n d'une polyamine primaire R(-X-R1-NH₂)ₙ qui est une diamine ou une triamine primaire
- avec chaque groupement d'amine primaire -NH₂ étant un groupement terminal porté par un segment bivalent de chaîne oligomère R1 choisi parmi polyéther et polyester alkoxylé, de préférence polyéther et plus préférentiellement polyoxypropylène ou copolymères oxypropylène/oxyéthylène à majorité de motifs oxypropylène
- R : résidu alkylène en C₃-C₁₀ de valence n issu d'un polyol R(OH)ₙ ou d'une polyamine R(NH₂)ₙ ou R(NH-R3)ₙ, de préférence d'un polyol R(OH)ₙ
- X : O, NH ou NR3, de préférence O
- R2 étant le résidu gras en C₁₂-C₅₂, de préférence en C₁₆-C₃₆, plus préférentiellement en C₁₆-C₂₄, d'acide gras hydroxylé R2CO₂H, en particulier saturé et linéaire
- R2' étant le résidu d'acide monocarboxylique R2'CO₂H en C₂ à C₁₀, de préférence de C₂ à C₈, et porteur d'au moins un groupement hydroxy, de préférence d'au moins deux groupements hydroxy
- y représentant la fraction molaire moyenne de R2'CO₂H par rapport à la somme R2'CO₂H + R2CO₂H, dans ledit diamide avec y variant de 0,05 à 0,50, de préférence de 0,10 à 0,40, avec R2CO₂H et/ou R2'CO₂H pouvant être des mélanges d'acides respectifs,
- R3 étant un substituant alkyl en C₁-C₂
ou par
B) selon la formule (II) suivante dans le cas où ledit amide est un diamide :
(R2CONH)_{(1-y)-}R'-O-[CH₂-CH(R4)-O]ₓ-CH₂-CH(R4)-(NHCOR2')_{y} (II)
avec R' étant le résidu du monopropylène glycol sans OH : -CH(CH₃)-CH₂-
et R2 et R2' et y, étant définis comme dans la formule (I) ci-dessus et
x étant le nombre d'unités oxyalkylène -CH₂-CH(R4)-O- et pouvant varier de 5 à 45 de préférence de 5 à 40 et plus préférentiellement de 5 à 35,
R4 étant H ou méthyl avec l'unité répétitive oxyalkylène -CH₂-CH(R4)-O- étant éthoxy pour R4 étant H et propoxy pour R4 étant méthyl ou R4 correspond à un mélange éthoxy/propoxy et de préférence R4 est méthyl avec ladite unité oxyalkylène étant propoxy
et ledit amide ayant un point de fusion signifiant température de fusion, mesurée par DSC après deux passages à 10°C/min, allant de 10 à 110°C, de préférence de 20 à 100°C.

2. Amide gras selon la revendication 1, **caractérisé en ce que** la masse moléculaire moyenne en nombre Mn dudit amide gras défini selon A) formule (I), mesurée par GPC dans le THF en équivalents polystyrène, varie pour :
- n = 2 de 800 à 4000, de préférence de 1000 à 3800
- n = 3 de 1000 à 6000, de préférence de 2000 à 5500.

3. Amide gras selon la revendication 1 ou 2, **caractérisé en ce que** ledit segment de chaîne oligomère R1 pour l'amide gras selon A) formule (I), est un segment de chaîne polyéther, en particulier un segment de chaîne polyoxypropylène.

4. Amide gras selon l'une des revendications 1 à 3, **caractérisé en ce que** ledit segment de chaîne oligomère R1 a une masse moléculaire moyenne en nombre Mn allant de 400 à 2000, de préférence de 500 à 1500.

5. Amide gras selon l'une des revendications 1 à 4, **caractérisé en ce que** ledit acide gras hydroxylé R2CO₂H est sélectionné parmi l'acide 12-hydroxy stéarique (12-HSA) ; l'acide 9- ou 10- hydroxy stéarique (9-HSA ou 10-HSA), de préférence un mélange d'acide 9- et 10- hydroxy stéarique ; l'acide 14-hydroxy eicosanoïque (14-HEA) ; et leurs mélanges ; en particulier l'acide 12-hydroxy stéarique.

6. Amide gras selon l'une des revendications 1 à 5, **caractérisé en ce que** ledit amide est un diamide selon A) ou B) ou un triamide selon A) avec y/(1-y) variant de 1/20 à 1/2 et de préférence de 1/10 à 4/10.

7. Amide gras selon l'une des revendications 1 à 6, **caractérisé en ce que** ledit amide est un triamide selon A) avec deux résidus R2 issus d'acide gras hydroxylé R2CO₂H et 1 issu d'acide R2'CO₂H.

8. Composition de formulation d'un liant organique **caractérisée en ce qu'**elle comprend :
a) au moins un liant organique
b) au moins un amide gras tel que défini selon l'une des revendications 1 à 7, en particulier en tant qu'additif rhéologique.

9. Composition selon la revendication 8, **caractérisée en ce que** ledit liant a) est sélectionné parmi : les résines polysiloxanes terminées par des groupements silanes bloqués, les résines polyéthers terminées par des groupements silanes bloqués, les résines polysulfides terminées par des groupements silanes bloqués, les résines prépolymères polyuréthanes terminés par des groupements isocyanates, les résines PVC pour plastisols, les résines époxy porteuses de groupements époxy.

10. Composition selon la revendication 8 ou 9, **caractérisée en ce qu'**elle comprend en plus de a) et b) et en fonction dudit liant un plastifiant ou un diluant réactif comme défini ci-dessous :
c) un plastifiant pour les résines polysiloxanes, prépolymères polyuréthanes et résines PVC pour plastisols ou
d) un diluant réactif parmi monomères époxydés pour les résines époxy et en option
e) pour les systèmes deux composants, un durcisseur pour les résines époxy ou polyuréthane.

11. Composition selon la revendication 10, **caractérisée en ce que** ledit liant organique a) est une résine polysiloxane, une résine prépolymère polyuréthane ou une résine PVC pour plastisol et **en ce que** ledit plastifiant est sélectionné parmi : les phtalates, les adipates, les trimellitates, les sebacates, les benzoates, les citrates, les phosphates, les époxydes, les polyesters les esters alkyl-sulfonates et non-phtalates substituts de phtalates.

12. Utilisation d'au moins un amide gras tel que défini selon l'une des revendications 1 à 7, **caractérisée en ce que** ledit amide est utilisé comme additif de rhéologie, en particulier comme agent thixotrope.

13. Utilisation selon la revendication 12, **caractérisée en ce qu'**il s'agit d'utilisation dans des compositions de revêtements, d'adhésifs, de plastisols PVC ou de mastics, de préférence des compositions de plastisols PVC et des compositions de mastics.

14. Utilisation selon la revendication 13, **caractérisée en ce qu'**il s'agit d'utilisation dans des compositions de mastics réticulables par l'humidité à base de résines polysiloxanes terminées par des groupements silanes bloqués, les résines polyéthers terminées par des groupements silanes bloqués, les résines polysulfides terminées par des groupements silanes bloqués, en particulier silanes bloqués par groupements alkoxys ou des résines prépolymères polyuréthanes terminées par des groupements isocyanates.

15. Revêtement, en particulier de plastisol PVC, joint d'adhésif ou de mastic, **caractérisé en ce qu'**il résulte de l'utilisation d'au moins un amide gras tel que défini selon l'une des revendications 1 à 7, comme additif de rhéologie, en particulier comme agent thixotrope.

## Patentansprüche

1. Multifunktionelles Fettamid, **dadurch gekennzeichnet, dass** es sich um ein Diamid oder ein Triamid oder eine Mischung davon handelt und dass das Fettamid dargestellt wird durch:
A) gemäß folgender Formel (I):
R[(-X-R1-NHCO-R2)_{n(1-y)}] [(-X-R1-NHCO-R2')_{ny}] (I)
wobei
- n für 2 oder 3, vorzugsweise 3, steht,
- R(-X-R1-)ₙ für den n-wertigen Rest eines primären Polyamins R(-X-R1-NH₂)ₙ, bei dem es sich um ein Diamin oder ein primäres Triamin handelt, steht,
- jede primäre Amingruppe -NH₂ eine Endgruppe an einem zweiwertigen Oligomerkettensegment R1 ist, welches aus Polyether und alkoxyliertem Polyester, vorzugsweise Polyether und weiter bevorzugt Polyoxypropylen oder Oxypropylen/Oxyethylen-Copolymeren mit einem Hauptanteil von Oxypropyleneinheiten ausgewählt ist,
- R: n-wertiger C₃-C₁₀-Alkylenrest, der sich von einem Polyol R(OH)ₙ oder von einem Polyamin R(NH₂)ₙ oder R(NH-R3)ₙ, vorzugsweise von einem Polyol R(OH)ₙ, ableitet,
- X: O, NH oder NR3, vorzugsweise O,
- wobei R2 für den C₁₂-C₅₂-, vorzugsweise C₁₆-C₃₆- und weiter bevorzugt C₁₆-C₂₄-Fettsäurerest einer hydroxylierten Fettsäure R2CO₂H, insbesondere gesättigt und linear, steht,
- R2' für den Rest einer C₂- bis C₁₀- und vorzugsweise C₂-bis C₈-Monocarbonsäure R2'CO₂H mit mindestens einer Hydroxygruppe, vorzugsweise mindestens zwei Hydroxygruppen, steht,
- y den durchschnittlichen molaren Anteil von R2'CO₂H in Bezug auf die Summe von R2'CO₂H + R2CO₂H in dem Diamid darstellt, wobei y zwischen 0,05 und 0,50, vorzugsweise zwischen 0,10 und 0,40, variiert, wobei es sich bei R2CO₂H und/oder R2'CO₂H um Gemische der jeweiligen Säuren handeln kann,
- R3 für einen C₁-C₂-Substituenten steht,
oder durch
B) gemäß folgender Formel (II) in dem Fall, dass es sich bei dem Amid um ein Diamid handelt:
(R2CONH)_{(1-y)}-R'-O-[CH₂-CH(R4)-O]ₓ-CH₂-CH(R4)-(NHCOR2')_{y} (II)
wobei R' für den Rest von Monopropylenglykol ohne OH steht: -CH(CH₃)-CH₂-
und R2 und R2' und y wie in obiger Formel (I) definiert sind und
x für die Zahl der Oxyalkyleneinheiten -CH₂-CH(R4)-O-steht und von 5 bis 45, vorzugsweise von 5 bis 40 und weiter bevorzugt von 5 bis 35 variieren kann,
R4 für H oder Methyl steht, wobei es sich bei der Oxyalkylen-Wiederholungseinheit -CH₂-CH(R4)-O- um Ethoxy handelt, wenn R4 für H steht, und um Propoxy handelt, wenn R4 für Methyl steht, oder R4 einem Ethoxy/Propoxy-Gemisch entspricht und vorzugsweise R4 für Methyl steht, wobei es sich bei der Oxyalkyleneinheit um Propoxy handelt,
und das Amid einen Schmelzpunkt, d. h. eine mittels DSC nach zwei Durchgängen bei 10 °C/min gemessene Schmelztemperatur, im Bereich von 10 bis 110 °C, vorzugsweise von 20 bis 100 °C, aufweist.

2. Fettamid nach Anspruch 1, **dadurch gekennzeichnet, dass** die mittels GPC in THF in Polystyroläquivalenten gemessene zahlenmittlere Molmasse Mn des gemäß A) Formel (I) definierten Fettamids für:
- n = 2 von 800 bis 4000, vorzugsweise von 1000 bis 3800,
- n = 3 von 1000 bis 6000, vorzugsweise von 2000 bis 5500, variiert.

3. Fettamid nach Anspruch 1 oder 2, **dadurch gekennzeichnet, dass** es sich bei dem Oligomerkettensegment R1 für das Fettamid gemäß A) Formel (I) um ein Polyetherkettensegment, insbesondere ein Polyoxypropylenkettensegment, handelt.

4. Fettamid nach einem der Ansprüche 1 bis 3, **dadurch gekennzeichnet, dass** das Oligomerkettensegment R1 eine zahlenmittlere Molmasse Mn im Bereich von 400 bis 2000, vorzugsweise von 500 bis 1500, aufweist.

5. Fettamid nach einem der Ansprüche 1 bis 4, **dadurch gekennzeichnet, dass** die hydroxylierte Fettsäure R2CO₂H aus 12-Hydroxystearinsäure (12-HSA); 9- oder 10-Hydroxystearinsäure (9-HSA oder 10-HSA), vorzugsweise einem Gemisch von 9- und 10-Hydroxystearinsäure; 14-Hydroxyeicosansäure (14-HEA) und Mischungen davon; insbesondere 12-Hydroxystearinsäure, ausgewählt ist.

6. Fettamid nach einem der Ansprüche 1 bis 5, **dadurch gekennzeichnet, dass** es sich bei dem Amid um ein Diamid gemäß A) oder B) oder ein Triamid gemäß A), wobei y/(1-y) zwischen 1/20 und 1/2 und vorzugsweise zwischen 1/10 und 4/10 variiert, handelt.

7. Fettamid nach einem der Ansprüche 1 bis 6, **dadurch gekennzeichnet, dass** es sich bei dem Amid um ein Triamid nach A) mit zwei R2-Resten, die sich von einer hydroxylierten Fettsäure R2CO₂H ableiten, und 1, der sich von einer Säure R2'CO₂H ableitet, handelt.

8. Formulierungszusammensetzung eines organischen Bindemittels, **dadurch gekennzeichnet, dass** sie Folgendes umfasst:
a) mindestens ein organisches Bindemittel,
b) mindestens ein Fettamid gemäß einem der Ansprüche 1 bis 7, insbesondere als Rheologieadditiv.

9. Zusammensetzung nach Anspruch 8, **dadurch gekennzeichnet, dass** das Bindemittel a) ausgewählt ist aus: Polysiloxanharzen mit blockierten Silanendgruppen, Polyetherharzen mit blockierten Silanendgruppen, Polysulfidharzen mit blockierten Silanendgruppen, Polyurethanprepolymerharzen mit Isocyanatendgruppen, PVC-Harzen für Plastisole, Epoxidharzen mit Epoxidgruppen.

10. Zusammensetzung nach Anspruch 8 oder 9, **dadurch gekennzeichnet, dass** sie neben a) und b) und in Abhängigkeit von dem Bindemittel einen Weichmacher oder ein reaktives Verdünnungsmittel gemäß nachstehender Definition umfasst:
c) einen Weichmacher für Polysiloxanharze, Polyurethanprepolymere und PVC-Harze für Plastisole oder
d) ein reaktives Verdünnungsmittel aus epoxidierten Monomeren für Epoxidharze und gegebenenfalls
e) für Zweikomponentensysteme einen Härter für Epoxid- oder Polyurethanharze.

11. Zusammensetzung nach Anspruch 10, **dadurch gekennzeichnet, dass** es sich bei dem organischen Bindemittel a) um ein Polysiloxanharz, ein Polyurethanprepolymerharz oder ein PVC-Harz für Plastisole handelt und dass der Weichmacher ausgewählt ist aus: Phthalaten, Adipaten, Trimellitaten, Sebacaten, Benzoaten, Citraten, Phosphaten, Epoxiden, Polyestern, Alkylsulfonatestern und Nicht-Phthalat-Ersatzstoffen für Phthalate.

12. Verwendung von mindestens einem Fettamid nach einem der Ansprüche 1 bis 7, **dadurch gekennzeichnet, dass** das Amid als Rheologieadditiv, insbesondere als Thixotropiermittel, verwendet wird.

13. Verwendung nach Anspruch 12, **dadurch gekennzeichnet, dass** es sich um eine Verwendung in Beschichtungs-, Klebstoff-, PVC-Plastisol- oder Kittzusammensetzungen, vorzugsweise PVC-Plastisolzusammensetzungen und Kittzusammensetzungen, handelt.

14. Verwendung nach Anspruch 13, **dadurch gekennzeichnet, dass** es sich um eine Verwendung in feuchtigkeitsvernetzbaren Kittzusammensetzungen auf Basis von Polysiloxanharzen mit blockierten Silanendgruppen, Polyetherharzen mit blockierten Silanendgruppen, Polysulfidharzen mit blockierten Silanendgruppen, insbesondere mit Alkoxygruppen blockierten Silanen oder Polyurethanprepolymerharzen mit Isocyanatendgruppen, handelt.

15. Beschichtung, insbesondere PVC-Plastisol-Beschichtung, Klebstoff- oder Kittfuge, **dadurch gekennzeichnet, dass** sie aus der Verwendung von mindestens einem Fettamid gemäß einem der Ansprüche 1 bis 7 als Rheologieadditiv, insbesondere als Thixotropiermittel, resultiert.

## Claims

1. Polyfunctional fatty amide, **characterized in that** it is a diamide or a triamide or a mixture thereof and that said fatty amide is represented by:
A) according to the following formula (I):
R[(-X-R1-NHCO-R2)_{n(1-y)}] [(-X-R1-NHCO-R2')_{ny}] (I)
with
- n being 2 or 3, preferably 3,
- R(-X-R1-)ₙ being the residue of valency n of a primary polyamine R(-X-R1-NH₂)ₙ which is a primary diamine or triamine,
- with each primary amine group -NH₂ being a terminal group borne by a bivalent oligomer chain segment R1 chosen from alkoxylated polyester and polyether, preferably polyether and more preferentially polyoxypropylene or oxypropylene/oxyethylene copolymers having a predominance of oxypropylene units,
- R: C₃-C₁₀ alkylene residue of valency n resulting from a polyol R(OH)ₙ or from a polyamine R(NH₂)ₙ or R(NH-R3)ₙ, preferably from a polyol R(OH)ₙ,
- X: O, NH or NR3, preferably O,
- R2 being the C₁₂-C₅₂, preferably C₁₆-C₃₆, more preferentially C₁₆-C₂₄, fatty residue of hydroxylated fatty acid R2CO₂H, in particular saturated and linear,
- R2' being the C₂ to C₁₀, preferably C₂ to C₈, monocarboxylic acid R2'CO₂H residue bearing at least one hydroxyl group, preferably at least two hydroxyl groups,
- y representing the mean molar fraction of R2'CO₂H relative to the sum of R2'CO₂H + R2CO₂H, in said diamide, with y varying from 0.05 to 0.50, preferably from 0.10 to 0.40, with it being possible for R2CO₂H and/or R2'CO₂H to be mixtures of respective acids,
- R3 being a C₁-C₂ alkyl substituent,
or by
B) according to the following formula (II) in the case in which said amide is a diamide:
(R2CONH)_{(1-y)}-R'-O-[CH₂-CH(R4)-O]ₓ-CH₂-CH(R4)-(NHCOR2')_{y} (II)
with R' being the residue of monopropylene glycol without OH: -CH(CH₃)-CH₂-
and R2 and R2' and y being defined as in formula (I) above, and
x being the number of oxyalkylene units -CH₂-CH(R4)-O- and it being possible for x to vary from 5 to 45,
preferably from 5 to 40 and more preferentially from 5 to 35,
R4 being H or methyl with the repeating oxyalkylene unit -CH₂-CH(R4)-O- being ethoxy when R4 is H and propoxy when R4 is methyl or R4 corresponds to an ethoxy/propoxy mixture and preferably R4 is methyl with said oxyalkylene unit being propoxy,
and said amide having a melting point, meaning melting temperature, measured by DSC after two passes at 10°C/min, ranging from 10 to 110°C, preferably from 20 to 100°C.

2. Fatty amide according to Claim 1, **characterized in that** the number-average molecular weight Mn of said fatty amide defined according to A) formula (I), measured by GPC in THF as polystyrene equivalents varies for:
- n = 2 from 800 to 4000, preferably from 1000 to 3800,
- n = 3 from 1000 to 6000, preferably from 2000 to 5500.

3. Fatty amide according to Claim 1 or 2, **characterized in that** said oligomer chain segment R1 for the fatty amide according to A) formula (I) is a polyether chain segment, in particular a polyoxypropylene chain segment.

4. Fatty amide according to one of Claims 1 to 3, **characterized in that** said oligomer chain segment R1 has a number-average molecular weight Mn ranging from 400 to 2000, preferably from 500 to 1500.

5. Fatty amide according to one of Claims 1 to 4, **characterized in that** said hydroxylated fatty acid R2CO₂H is selected from 12-hydroxystearic acid (12-HSA); 9- or 10-hydroxystearic acid (9-HSA or 10-HSA), preferably a mixture of 9- and 10-hydroxystearic acids; 14-hydroxyeicosanoic acid (14-HEA); and mixtures thereof; in particular 12-hydroxystearic acid.

6. Fatty amide according to one of Claims 1 to 5, **characterized in that** said amide is a diamide according to A) or B) or a triamide according to A) with y/(1-y) varying from 1/20 to 1/2 and preferably from 1/10 to 4/10.

7. Fatty amide according to one of Claims 1 to 6, **characterized in that** said amide is a triamide according to A) with two R2 residues resulting from hydroxylated fatty acid R2CO₂H and 1 resulting from acid R2'CO₂H.

8. Formulation composition of an organic binder, **characterized in that** it comprises:
a) at least one organic binder,
b) at least one fatty amide as defined in one of Claims 1 to 7, in particular as rheological additive.

9. Composition according to Claim 8, **characterized in that** said binder a) is selected from: polysiloxane resins terminated by blocked silane groups, polyether resins terminated by blocked silane groups, polysulfide resins terminated by blocked silane groups, polyurethane prepolymer resins terminated by isocyanate groups, PVC resins for plastisols, epoxy resins bearing epoxy groups.

10. Composition according to Claim 8 or 9, **characterized in that** it comprises, in addition to a) and b) and depending on said binder, a plasticizer or a reactive diluent as defined below:
c) a plasticizer for polysiloxane resins, polyurethane prepolymer resins and PVC resins for plastisols or
d) a reactive diluent from epoxidized monomers for epoxy resins and optionally
e) for two-component systems, a hardener for the epoxy or polyurethane resins.

11. Composition according to Claim 10, **characterized in that** said organic binder a) is a polysiloxane resin, a polyurethane prepolymer resin or a PVC resin for plastisols and **in that** said plasticizer is selected from: phthalates, adipates, trimellitates, sebacates, benzoates, citrates, phosphates, epoxides, polyesters, alkylsulfonate esters and non-phthalate substitutes for phthalates.

12. Use of at least one fatty amide as defined in one of Claims 1 to 7, **characterized in that** said amide is used as rheology additive, in particular as thixotropic agent.

13. Use according to Claim 12, **characterized in that** it concerns use in coating, adhesive, PVC plastisol or mastic compositions, preferably PVC plastisol compositions and mastic compositions.

14. Use according to Claim 13, **characterized in that** it concerns use in mastic compositions which can be crosslinked by moisture based on polysiloxane resins terminated by blocked silane groups, polyether resins terminated by blocked silane groups, polysulfide resins terminated by blocked silane groups, in particular silanes blocked by alkoxy groups, or polyurethane prepolymer resins terminated by isocyanate groups.

15. Coating, in particular PVC plastisol coating, adhesive seal or mastic seal, **characterized in that** it results from the use of at least one fatty amide as defined in one of Claims 1 to 7, as rheology additive, in particular as thixotropic agent.
